# EUROPÄISCHE PATENTANMELDUNG

(11) **EP 1 340 747 A1**
(43) Veröffentlichungstag der Anmeldung: **03.09.2003**
(21) Anmeldenummer: 03003561.2
(22) Anmeldetag: 17.02.2003
(51) Int. Cl.: C07D 213/79, C07D 213/80

(54) **Verfahren zur Herstellung von 2-Halogenalkyl-Nicotinsäurealkylester**

(30) Priorität: 28.02.2002 DE 10208955
(71) Anmelder: BAYER AG, 51368 Leverkusen (DE)
(72) Erfinder: Müller, Peter, Dr., 51519 Odenthal (DE)

(57) **Zusammenfassung**

Die vorliegende Erfindung betrifft ein Verfahren zur Herstellung von 2-Halogenalkyl-Nicotinsäurenalkylester der Formel (IV), ausgehend von halogenmethylsubstituierten Enonen und 3-Dialkylaminoacrylsäureestern sowie Zwischenverbindungen der Formel C (III) des erfindungsgemäßen Verfahrens. 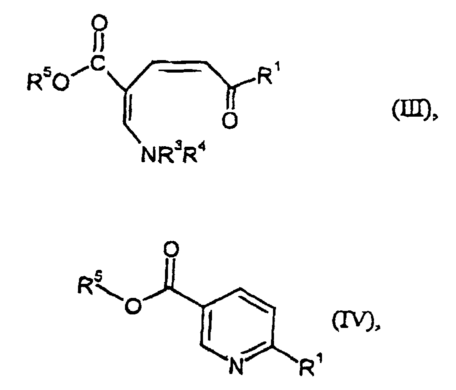 in denen R¹ für C₁-C₁₂-Halogenalkyl steht und R³, R⁴ und R⁵ für C₁-C₁₂-Alkyl stehen.

## Beschreibung

Die vorliegende Erfindung betrifft ein Verfahren zur Herstellung von 2-Halogen-alkylnicotinsäure und Derivaten davon sowie Zwischenverbindungen.

2-Halogenalkylnicotinsäuren und deren Derivate, insbesondere sei 2-Trifluormethylnicotinsäure genannt, sind wertvolle Zwischenprodukte z.B. für die Herstellung von pharmazeutischen Wirkstoffen und Agrochemikalien.

Aus Tetrahedron Lett. 1998, 39, 7965 und J. Heterocycl. Chem. 1995, 32, 543 ist bekannt, dass man aus halogenmethylsubstituierten Enonen und Enaminonitrilen 2-Halogenmethyl-5-cyanopyridine herstellen kann. Diese können dann in an sich bekannter Weise, z.B. durch Verseifung, zu den entsprechende Nicotinsäuren umgesetzt werden. Nachteilig für eine industrielle Anwendung dieses Verfahrens ist, dass die entsprechenden Enaminonitrile aufwändig herzustellen und daher teuer sind.

Gemäß Heterocycles 1997, 46, 129 kann man aus β-Trifluoracetylvinylamin und substituierten 1,3-Diketonen 2-Trifluormethylnicotinsäuren herstellen. Allerdings sind nach diesem Verfahren in der Regel nur in 6-Position substituierte Nicotinsäuren zugänglich, aus denen die in 6-Position unsubstituierten Verbindungen z.B. durch reduktive Dehalogenierung der 6-Halogenverbindungen zugänglich sind. Wegen der hohen Zahl an Reaktionsschritten ist auch dieses Verfahren unwirtschaftlich.

Alternativ dazu können nach WO 00/39094 substituierte β-Acetylvinylamine und β-Ketoester in die entsprechenden Nicotinsäureester überführt werden. Auch dieses Verfahren ist für die industrielle Anwendung wegen der aufwändigen Herstellung der isolierten β-Acetylvinylamine ungeeignet.

Es bestand daher das Bedürfnis, ein Verfahren zu entwickeln, das - ausgehend von leicht verfügbaren Edukten - in wenigen Schritten die Herstellung von 2-Halogenalkylnicotinsäuren ermöglicht.

Es wurde nun ein Verfahren zur Herstellung von 2-Halogenalkylnicotinsäuren und 2-Halogenalkylsäurederivaten gefunden, das dadurch gekennzeichnet ist, dass
a) Verbindungen der allgemeinen Formel (I) 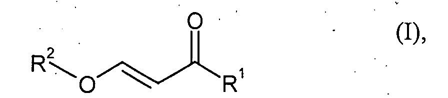 in der
   - R¹: für C₁-C₁₂-Halogenalkyl steht und
   - R²: für C₁-C₁₂-Alkyl steht,
   mit Verbindungen der allgemeinen Formel (II) 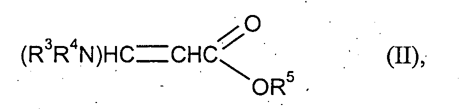 in der
   R³, R⁴ und R⁵ jeweils unabhängig voneinander für C₁-C₁₂-Alkyl stehen,
   zu Verbindungen der allgemeinen Formel (III) umgesetzt werden 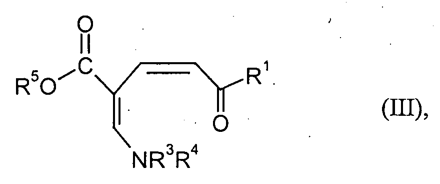 in der
   R¹, R³, R⁴ und R⁵ die oben genannte Bedeutung besitzen, und
b) die Verbindungen der allgemeinen Formel (III) mit Ammoniak oder Ammoniumsalzen zu Verbindungen der allgemeinen Formel (IV) umgesetzt werden 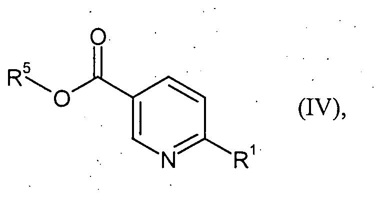 in der
   R¹ und R⁵ die oben genannte Bedeutung besitzen, und
c) gegebenenfalls die Verbindungen der allgemeinen Formel (IV)
   zu Verbindungen der allgemeinen Formel (V) 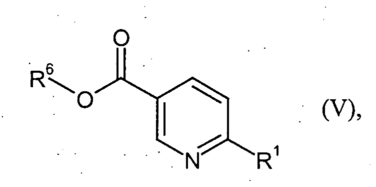 in der
   - R¹: für C₁-C₁₂-Halogenalkyl steht und
   - R⁶: für M oder Wasserstoff steht, wobei M für ein Äquivalent eines Alkalimetalls oder ein halbes Äquivalent eines Erdalkalimetalls steht,
   verseift werden.

Vom Rahmen der Erfindung sind auch die Verbindungen der allgemeinen Formeln (III) und (IV) selbst umfasst.

Im Rahmen der Erfindung bedeutet Alkyl einen geradkettigen oder cyclischen, verzweigten oder unverzweigten Alkylrest. Beispielsweise und bevorzugt steht C₁-C₁₂-Alkyl für Methyl, Ethyl, n-Propyl, Isopropyl, n-Butyl, tert.-Butyl, n-Pentyl, Cyclohexyl, n-Hexyl, n-Octyl, iso-Octyl, n-Decyl und n-Dodecyl.

Im Rahmen der Erfindung bedeutet Halogenalkyl einen geradkettigen oder cyclischen, verzweigten oder unverzweigten Alkylrest, der insgesamt einfach oder mehrfach, in α-Stellung mindestens einfach durch Halogenatome substituiert ist, die unabhängig voneinander ausgewählt sein können aus der Gruppe Fluor, Chlor und Brom.

C₁-C₁₂-Halogenalkyl steht beispielsweise und bevorzugt für Fluormethyl, Difluormethyl, Trifluormethyl, Tribrommethyl, Dibromfluormethyl, Bromdifluormethyl, Trichlormethyl, Dichlorfluormethyl, Chlordifluormethyl, Trifluormethyl, Tribrommethyl, Dibromfluormethyl, Pentafluorethyl und n-Nonafluorbutyl.

Es sei darauf hingewiesen, dass vom Rahmen der Erfindung auch beliebige Kombinationen von Vorzugsbereichen mitumfasst sind.

Besonders bevorzugt steht in den Verbindungen der allgemeinen Formel (I) R¹ für C₁-C₄-Halogenalkyl, ganz besonders bevorzugt für Trifluormethyl, Trichlormethyl, Dichlorfluormethyl und Pentafluorethyl, wobei Trifluormethyl noch weiter bevorzugt ist.

Besonders bevorzugt steht in den Verbindungen der allgemeinen Formel (I) R² für C₁-C₄-Alkyl, ganz besonders bevorzugt für Ethyl oder Methyl, wobei Ethyl noch weiter bevorzugt ist.

Als Verbindungen der allgemeinen Formel (I) seien insbesondere genannt: 1,1,1 -Trifluor-2-oxo-4-ethoxy-but-3-en, 1 -Brom-1,1-difluor-2-oxo-4-ethoxy-but-3-en, 1-Chlor-1,1-difluor-2-oxo-4-ethoxy-but-3-en und 1,1,1-Trichlor-2-oxo-4-ethoxy-but-3-en.

Besonders bevorzugt stehen in den Verbindungen der allgemeinen Formel (II) R³ und R⁴ jeweils identisch für C₁-C₄-Alkyl, ganz besonders bevorzugt jeweils identisch für Methyl oder Ethyl, wobei Methyl noch weiter bevorzugt ist.

Besonders bevorzugt steht in den Verbindungen der allgemeinen Formel (II) R⁵ für C₁-C₄-Alkyl, ganz besonders bevorzugt für Ethyl oder Methyl.

Als Verbindungen der allgemeinen Formel (II) seien insbesondere genannt: 3-N,N-Dimethylamino-acrylsäuremethylester, 3-N,N-Diethylamino-acrylsäureethylester, 3-N,N-Dimethylamino-acrylsäureethylester und 3-N,N-Diethylamino-acryl-säuremethylester.

Die Verbindungen der allgemeinen Formel (I) sind kommerziell verfügbar oder nach literaturbekannten Methoden oder analog dazu synthetisierbar.

Die Verbindungen der allgemeinen Formel (II) sind ebenfalls kommerziell verfügbar oder nach literaturbekannten Methoden (siehe z.B. WO 00/000460 oder DE-OS 44 18 155) oder analog dazu synthetisierbar.

Die Umsetzung gemäß Schritt a) wird vorzugsweise in Gegenwart von Lösungsmittel durchgeführt.

Als Lösungsmittel eignen sich beispielsweise dipolare, aprotische Lösungsmittel und Mischungen, die dipolare, aprotische Lösungsmittel enthalten.

Geeignete dipolare, aprotische Lösungsmittel sind beispielsweise Nitrile wie Acetonitril, Propionitril, n- und i-Butyronitril, Benzylnitril und Benzonitril, Amide wie Formamid, N-Methylformamid, N,N-Dimethylformamid, N,N-Dimethylacetamid und N-Methylpyrrolidon, Ester wie Methyl-, Ethyl- oder Butylacetat, Sulfoxide wie Dimethylsulfoxid und Sulfone wie Sulfolan oder Mischungen davon.

Die dipolaren, aprotischen Lösungsmittel können auch in Mischungen beispielsweise mit aliphatischen und/oder aromatischen Kohlenwasserstoffen und/oder Ethern eingesetzt werden.

Besonders bevorzugte dipolare, aprotische Lösungsmittel sind N,N-Dimethylform-amid und N,N-Dimethylacetamid.

Ganz besonders bevorzugt ist N,N-Dimethylformamid.

Die Menge von eingesetztem Lösungsmittel ist nicht kritisch, bevorzugt setzt man pro mol der jeweiligen Verbindung der allgemeinen Formel (I) 250 bis 500 ml ein.

Zur Durchführung des Schrittes a) kann man z. B. die jeweilige Verbindung der allgemeinen Formel (I) und die jeweilige Verbindung der allgemeinen Formel (II) in einem molaren Verhältnis von beispielsweise 0,3:1 bis 5:1, bevorzugt 0,5:1 bis 2:1, besonders bevorzugt 0,9 bis 1,1 und ganz besonders bevorzugt äquimolar einsetzen.

Die Reaktionstemperatur in Schritt a) kann beispielsweise 0 bis 100°C, bevorzugt 20 bis 80°C und besonders bevorzugt 30 bis 50°C betragen.

Die Reaktionszeit für Schritt a) kann beispielsweise 5 min bis 48 h betragen, bevorzugt sind 4 bis 12 h.

Schritt a) des erfindungsgemäßen Verfahrens kann beispielsweise bei einem Druck von 0,5 bis 100 bar durchgeführt werden, Umgebungsdruck ist bevorzugt.

Auf diese Weise werden Verbindungen der allgemeinen Formel (III) erhalten 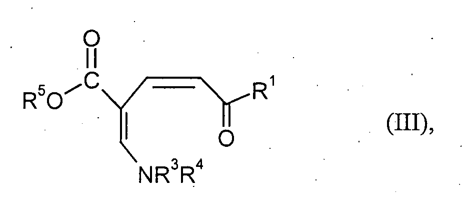 in denen die Reste R¹, R³, R⁴ und R⁵ die gleichen Bedeutungen und Vorzugsbereiche besitzen, die unter den allgemeinen Formeln (I) und (II) genannt worden sind.

Als Einzelverbindungen seien genannt:
2-Dimethylaminomethylen-6,6,6-trifluor-5-oxo-3-hexensäureethylester,2-Dimethyl-aminomethylen-6-brom-6,6-difluor-5-oxo-3-hexensäureethylester, 2-Dimethylamino-methylen-6-chlor-6,6-difluor-5-oxo-3-hexensäureethylester, 2-Dimethylamino-methylen-6,6,6-trichlor-5-oxo-3-hexensäureethylester.

Die Verbindungen der allgemeinen Formel (III) können entweder isoliert oder vorzugsweise direkt weiter umgesetzt werden.

Anschließend wird Schritt b) durchgeführt, die Umsetzung mit Ammoniak oder Ammoniumsalzen, bevorzugt Ammoniumsalzen. In einer bevorzugten Ausführungsform des erfindungsgemäßen Verfahrens wird für den Einsatz in Schritt b) direkt die Reaktionslösung aus Schritt a) verwendet. Alternativ dazu können auch isolierte Verbindungen der allgemeinen Formel (III) eingesetzt werden.

Bezogen auf die jeweils ursprünglich eingesetzte Verbindung der allgemeinen Formel (II) kann man z. B. 0,3 bis 10 mol, bevorzugt 0,9 bis 2,5 mol und besonders bevorzugt 1,0 bis 1,5 mol Ammoniak oder Ammoniumsalz einsetzen.

Besonders bevorzugt werden Ammoniumsalze der allgemeinen Formel (VI) eingesetzt

NH₄X (VI),

in der
- X: für ein Monoanion einer anorganischen oder organischen Säure steht oder Mischungen solcher Ammoniumsalze.

Bevorzugt steht X für ein Halogenid oder ein Monoanion einer Carbonsäure.

Besonders bevorzugt steht X für Chlorid, Bromid oder Acetat, ganz besonders bevorzugt für Acetat.

Die Reaktionstemperatur in Schritt b) kann beispielsweise 0 bis 100°C, bevorzugt 20 bis 80°C und besonders bevorzugt 30 bis 50°C betragen.

Die Reaktionszeit für Schritt b) kann beispielsweise 5 min bis 48 h betragen, bevorzugt sind 30 min bis 4 h.

Schritt b) des erfindungsgemäßen Verfahrens kann beispielsweise bei einem Druck von 0,5 bis 100 bar durchgeführt werden, Umgebungsdruck ist bevorzugt.

Nach an sich bekannten Aufarbeitungsmethoden erhält man auf erfindungsgemäße Weise Verbindungen der allgemeinen Formel (IV), die gegebenenfalls Schritt c), der Verseifung, unterworfen werden können.

In der allgemeinen Formel (IV) besitzen R¹ und R⁵ die gleichen Bedeutungen und Vorzugsbereiche, die unter den allgemeinen Formeln (I) und (II) angegeben wurden.

Als Einzelverbindungen seien genannt:
2-Trifluormethyl-nicotinsäureethylester, 2-Bromdifluormethyl-nicotinsäurethylester, 2-Chlordifluormethyl-nicotinsäureethylester, 2-Trichlormethyl-nicotinsäureethylester.

Die Aufarbeitung kann in einer bevorzugten Ausführungsform des erfindungsgemäßen Verfahrens so durchgeführt werden, dass die gemäß Schritt b) erhaltene Reaktionslösung mit Wasser versetzt wird und die organische Phase oder nach mehrmaliger Extraktion mit einem mit Wasser nicht oder nur schwer mischbaren Lösungsmittel die organischen Phasen durch Verdampfen des Lösungsmittels eingeengt werden.

Mit Wasser nicht oder nur schwer mischbare Lösungsmittel sind beispielsweise aromatische Lösungsmittel wie z. B. Benzol, Toluol, o-, m-, p-Xylol, chlorierte Kohlenwasserstoffe wie z. B. Dichlormethan, Chloroform, Tetrachlorkohlenstoff, Ether wie z. B. Diethylether und tert.-Butylmethylether, Ester wie Methyl-, Ethyloder Butylacetat.

Die auf diese Weise erhaltenen Rohprodukte können beispielsweise durch Destillation bei Raumtemperatur, feste Produkte auch durch Kristallisation oder Sublimation weiter gereinigt werden. Man kann aber auch die Rohprodukte direkt verseifen.

Die Verseifung der Verbindung der allgemeinen Formel (IV) wird bevorzugt in Gegenwart von Basen durchgeführt.

Weiterhin ist der Einsatz von Lösungsmittel bei der Verseifung bevorzugt.

Als Lösungsmittel für Schritt c) sind beispielsweise geeignet:
Wasser, organische Lösungsmittel und Mischungen davon. Beispielhaft seien als organische Lösungsmittel genannt: aliphatische, alicyclische und aromatische Kohlenwasserstoffe wie Petrolether, Hexan, Heptan, Cyclohexan, Methylcyclohexan, Benzol, Toluol, Xylol und Decalin, Ether wie Diethyl-, Diisopropyl-, Methyl-t-butylund Methyl-t-amylether, Dioxan, Tetrahydrofuran, 1,2-Dimethoxyethan, 1,2-Diethoxyethan, Diethylenglykoldimethylether und Anisol und Alkohole wie Methanol, Ethanol, n- und i-Propanol, n-, iso-, sek.- und tert.-Butanol, Ethandiol, Propan-1,2-diol, Ethoxyethanol, Methoxyethanol, Diethylenglykolmonomethylether und Diethylenglykolmonoethylether. Bevorzugte Lösungsmittel sind Alkohole.

Besonders bevorzugt sind Ethanol oder Methanol.

Zur Verseifung können pro mol der Verbindungen der allgemeinen Formel (IV) z. B. 4Q bis 1000 ml, bevorzugt 90 bis 200 ml Lösungsmittel eingesetzt werden. Größere Mengen an Lösungsmittel sind möglich, aber unwirtschaftlich.

Als Basen werden beispielsweise und bevorzugt Alkalimetallhydroxide wie z.B. Lithiumhydroxid, Natriumhydroxid, Kaliumhydroxid beispielsweise in Form wässriger Lösungen oder Erdalkalimetallhydroxide wie z.B. Calciumhydroxid oder beliebige Mischungen davon eingesetzt.

Besonders bevorzugt ist Natriumhydroxid.

Zur Verseifung können pro mol der Verbindungen der allgemeinen Formel (IV) z. B. 0,5 bis 10 mol Base, bevorzugt 1,5 bis 2,5 mol Base eingesetzt werden.

Die Reaktionstemperatur für Schritt c) kann beispielsweise zwischen 0 und 200°C betragen, bevorzugt sind 20°C bis 120°C, besonders bevorzugt 50 bis 100°C.

Schritt c) des erfindungsgemäßen Verfahrens kann beispielsweise bei einem Druck von 0,5 bis 100 bar durchgeführt werden, Umgebungsdruck ist bevorzugt.

Auf erfindungsgemäße Weise erhält man gemäß Schritt c) Verbindungen der allgemeinen Formel (V), in der R² für M oder Wasserstoff steht.

Führt man die Verseifung, wie erfindungsgemäß bevorzugt, in Gegenwart von Base durch, so erhält man Verbindungen der allgemeinen Formel (V), in der M für das Kation der eingesetzten Base steht.

Diese können entweder isoliert oder durch Ansäuern in Verbindungen der allgemeinen Formel (I) überführt werden, in der R² für Wasserstoff steht.

Das Ansäuren kann beispielsweise mit Hilfe von sauren Salzen und/oder Säuren durchgeführt werden.

Geeignete Säuren sind beispielsweise und bevorzugt anorganische Säuren wie z.B. Salzsäure.

Die weitere Aufarbeitung und Isolierung der Reaktionsprodukte kann nach an sich bekannten Methoden erfolgen.

Verbindungen der allgemeinen Formel (V), in denen R² für Wasserstoff steht, können vorzugsweise durch Kristallisation, Destillation oder durch Entfernung der flüchtigen Bestandteile, gegebenenfalls im Vakuum, weiter gereinigt werden.

Die Verbindungen der allgemeinen Formeln (IV) und (V) eignen sich insbesondere zur Herstellung von Arzneimitteln und Agrochemikalien.

Das erfindungsgemäße Verfahren ermöglicht die vorteilhafte Herstellung von 2-Halogenalkylnicotinsäure und 2-Halogenalkylnicotinsäurederivaten in einem hocheffizienten Verfahren in sehr wenigen Stufen und ohne besondere sicherheitstechnische Vorkehrungen.

### Beispiele

### Beispiel 1

### Herstellung von 1,1,1 -Trifluormethyl-2-oxo-4-ethoxy-but-3-en

Zu 3000 g Trifluoressigsäureanhydrid in 4200 ml tert.-Butyl-methylether wurde bei -10°C bis 0°C ein Gemisch aus 1133 g Ethylvinylether und 1130 g Pyridin getropft. Die Mischung wurde anschließend über Nacht bis zum vollständigen Umsatz (GC) bei Raumtemperatur gerührt. Das Reaktionsgemisch wurde filtriert und das Filtrat mit einer Spatelspitze des Radikalfängers Junol versetzt und bei 40°C und einem Druck von 100 mbar eingeengt. Dieser Ansatz wurde zweimal wiederholt. Alle drei Ansätze wurden vereinigt (9024,5 g Rohmaterial) und unter Normaldruck an einer 1 m-Füllkörperkolonne destilliert. Es wurden 5960 g Produkt erhalten. Das Produkt war laut GC zu 97,9 % rein. Das entspricht einer Ausbeute von 81 % der Theorie.

### Beispiel 2

### Herstellung von 2-Trifluormethyl-nicotinsäureethylester

Zu einer Lösung aus 4500 g des gemäß Beispiel 1 erhaltenen Produktes in 9000 ml N,N-Dimethylformamid wurden bei 40°C langsam 3583,6 g Ethyl-3,3-dimethylaminoacrylat getropft und bis zum vollständigen Umsatz gerührt (12 h).

Die so erhaltene Lösung von 2-Dimethylaminomethylen-6,6,6-trifluor-5-oxo-3-hexensäureethylester wurde mit 2893,8 g Ammoniumacetat versetzt und wiederum bis zum vollständigen Umsatz gerührt (1,5 h). Das Reaktionsgemisch wurde mit 8 l Wasser verdünnt, die organische Phase abgetrennt und die wässrige Phase zweimal mit je 500 ml Toluol extrahiert. Die vereinigten organischen Phasen wurden eingeengt. Es wurden 8294,5 g Rohprodukt erhalten. Anschießend wurde das Rohmaterial unter vermindertem Druck destilliert. Es wurden 3402 g Produkt erhalten mit einem Siedepunkt von 64 bis 67°C bei 0,07 bis 0,09 mbar. Das Produkt war laut GC zu 94,4 % rein. Die Ausbeute betrug demnach 94 % der Theorie.

### Beispiel 3

### Herstellung von 2-Trifluormethyl-nicotinsäure

Eine Mischung aus 4782,5 g 25%ige Natronlauge, 1650 ml Ethanol und 3470 g des nach Beispiel 2 erhaltenen Produktes wurden 7 h unter Rückfluss (80°C) gerührt. Das Reaktionsgemisch wurde anschließend auf Eis gegeben und zweimal mit Dichlormethan extrahiert. Die organische Phase wurde abgetrennt und verworfen. Die wässrige Phase wurde mit konzentrierter Salzsäure auf einen pH-Wert von etwa 4 eingestellt. Anschließend wurde das ausgefallene Produkt abgesaugt. Das Filtrat wurde danach mit konzentrierter Salzsäure auf einen pH-Wert von etwa 2 eingestellt und abgesaugt. Die vereinigten Feststoffchargen wurden zweimal mit n-Hexan angeschlämmt, abgesaugt und getrocknet. Es wurden 2717,4 g Produkt erhalten. Das Produkt war laut GC zu über 99 % rein. Die Ausbeute betrug demnach 95 % der Theorie.

## Patentansprüche

1. Verfahren zur Herstellung von 2-Halogenalkylnicotinsäuren und 2-Halogen-alkylnicotinsäurederivaten, **dadurch gekennzeichnet, dass**
a) Verbindungen der allgemeinen Formel (I) 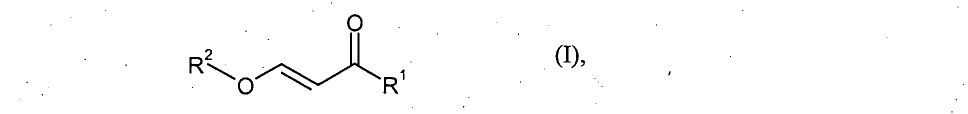 in der
R¹ für C₁-C₁₂-Halogenalkyl steht und
R² für C₁-C₁₂-Alkyl steht,
mit Verbindungen der allgemeinen Formel (II) 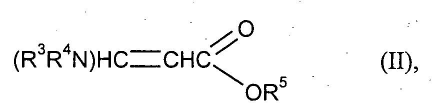 in der
R⁴, R⁵ und R⁶ jeweils unabhängig voneinander für C₁-C₁₂-Alkyl stehen,
zu Verbindungen der allgemeinen Formel (III) umgesetzt werden 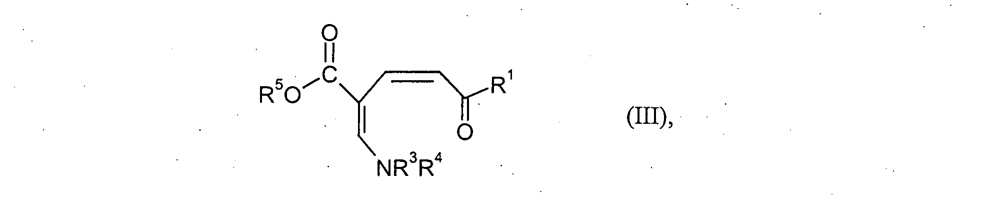 in der R¹, R⁴, R⁵ und R⁶ die oben genannte Bedeutung besitzen, und
b) die Verbindungen der allgemeinen Formel (III) mit Ammoniak oder Ammoniumsalzen zu Verbindungen der allgemeinen Formel (IV) umgesetzt werden 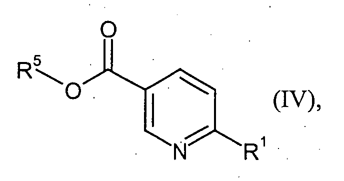 in der
R¹ und R⁵ die oben genannte Bedeutung besitzen.

2. Verfahren nach Anspruch 1, **dadurch gekennzeichnet, dass** als Verbindungen der allgemeinen Formel (I) solche eingesetzt werden, in denen R¹ für Fluormethyl, Difluormethyl, Trifluormethyl, Tribrommethyl, Dibromfluormethyl, Bromdifluormethyl, Trichlormethyl, Dichlorfluormethyl, Chlordifluormethyl, Trifluormethyl, Tribrommethyl, Dibromfluormethyl, Pentafluorethyl oder n-Nonafluorbutyl steht.

3. Verfahren nach einem oder mehreren der Ansprüche 1 bis 2, **dadurch gekennzeichnet, dass** als Verbindungen der allgemeinen Formel (I) solche eingesetzt werden, in denen R² für Ethyl oder Methyl steht.

4. Verfahren nach einem oder mehreren der Ansprüche 1 bis 3, **dadurch gekennzeichnet, dass** als Verbindungen der allgemeinen Formel (II) solche eingesetzt werden, in denen R³ und R⁴ jeweils identisch für C1-C₄-Alkyl stehen.

5. Verfahren nach einem oder mehreren der Ansprüche 1 bis 4, **dadurch gekennzeichnet, dass** als Verbindungen der allgemeinen Formel (II) solche eingesetzt werden, in denen R⁵ für C₁-C₄-Alkyl steht.

6. Verfahren nach einem oder mehreren der Ansprüche 1 bis 5, **dadurch gekennzeichnet, dass** Schritt a) in Gegenwart von Lösungsmittel durchgeführt wird.

7. Verfahren nach einem oder mehreren der Ansprüche 1 bis 6, **dadurch gekennzeichnet, dass** im Schritt a) die Verbindungen der allgemeinen Formel (I) und die Verbindungen der allgemeinen Formel (II) in einem molaren Verhältnis von 0,3:1 bis 5:1 eingesetzt werden.

8. Verfahren nach einem oder mehreren der Ansprüche 1 bis 7, **dadurch gekennzeichnet, dass** im Schritt a) die Reaktionstemperatur 0 bis 100°C beträgt.

9. Verfahren nach einem oder mehreren der Ansprüche 1 bis 8, **dadurch gekennzeichnet, dass** im Schritt b), bezogen auf die in Schritt a) eingesetzte Verbindung der allgemeinen Formel (I), 0,3 bis 10 mol Ammoniumsalz eingesetzt werden.

10. Verfahren nach einem oder mehreren der Ansprüche 1 bis 9, **dadurch gekennzeichnet, dass** im Schritt b) als Ammoniumsalze solche der allgemeinen Formel (VI) eingesetzt werden
NH₄X (VI),
in der X für ein Monoanion einer anorganischen oder organischen Säure steht oder Mischungen solcher Ammoniumsalze.

11. Verfahren nach einem oder mehreren der Ansprüche 1 bis 10, **dadurch gekennzeichnet, dass** im Schritt b) die Reaktionstemperatur 0 bis 100°C beträgt.

12. Verfahren nach einem oder mehreren der Ansprüche 1 bis 11, **dadurch gekennzeichnet, dass** als Schritt c) die Verseifung oder Verbindung der allgemeinen Formel (IV) zu Verbindungen der allgemeinen Formel (V) erfolgt, 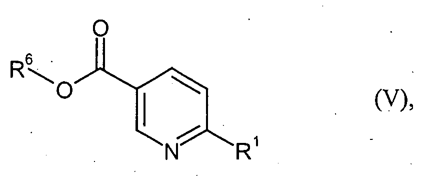 in der
R⁶ für Wasserstoff oder M und M für ein Äquivalent eines Alkalimetalls oder ein halbes Äquivalent eines Erdalkalimetalls steht.

13. Verfahren nach Anspruch 12, **dadurch gekennzeichnet, dass** Schritt c) in Gegenwart von Base durchgeführt wird.

14. Verfahren nach Anspruch 13, **dadurch gekennzeichnet, dass** zur Herstellung von Verbindungen der allgemeinen Formel (V) 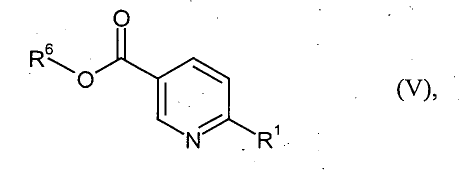 in der
M für Wasserstoff steht und anschließend an Schritt c) angesäuert wird.

15. Verbindungen der allgemeinen Formel (III) 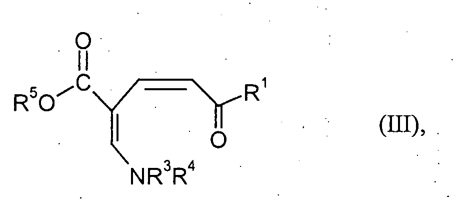 in der
R¹ für C₁-C₁₂-Halogenalkyl steht und
R³, R⁴ und R⁵ jeweils unabhängig voneinander für C₁-C₁₂-Alkyl stehen.

16. Verbindungen der allgemeinen Formel (IV) 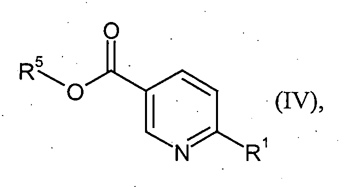 in der
R¹ für C₁-C₁₂-Halogenalkyl steht und
R⁵ für C₁-C₁₂-Alkyl steht.

17. Verwendung von Verbindungen gemäß einem oder mehreren der Ansprüche 15 bis 16 zur Herstellung von Arzneimitteln und Agrochemikalien oder Zwischenprodukten davon.

18. Verwendung von Verbindungen, die gemäß einem Verfahren nach einem oder mehreren der Ansprüche 1 bis 14 hergestellt wurden, zur Herstellung von Arzneimitteln und Agrochemikalien oder Zwischenprodukten davon.

19. Verwendung von Verbindungen gemäß Anspruch 15 zur Herstellung von Verbindungen gemäß Anspruch 16.
